# FASCICULE DE BREVET EUROPEEN

(11) **EP 0 530 060 B1**
(45) Date de publication et mention de la délivrance du brevet: **02.10.1996**
(21) Numéro de dépôt: 92402089.4
(22) Date de dépôt: 20.07.1992
(51) Int. Cl.: A61K 35/72

(54) **Médicament à base de dérivés soufrés neutralisés**
Arzneimittel auf neutralisierte Schwefelverbindungen-Basis
Medication based on neutralised sulphur derivatives

(30) Priorité: 01.08.1991 FR 9109792
(43) Date de publication de la demande: 03.03.1993
(73) Titulaire: Grimberg, Georges Serge, F-75007 Paris (FR)
(72) Inventeur: Grimberg, Georges Serge, F-75007 Paris (FR)
(74) Mandataire: Madeuf, René Louis

(56) Documents cités:
- EP-A- 0 113 608
- EP-A- 0 402 208
- FR-A- 2 228 470

## Description

Le soufre est l'un des constituants essentiels de la nature biologique et notamment de la peau humaine. Il entre, de plus, dans de nombreuses molécules médicamenteuses.

Les dérivés soufrés formés par S⁻⁻ et un cation, exemples Na₂S, sulfure de sodium ; K₂S, sulfure de potassium ; ZnS, sulfure de zinc ; Se₄S₄, sulfure de sélénium ; etc., ont en solution aqueuse une forte odeur d'hydrogène sulfuré H₂S et un pH alcalin.

Cependant, de nombreuses eaux thermales contiennent de faibles quantités de dérivés soufrés et sont utilisées pour le traitement des rhino-pharyngites en lavage nasal ou auriculaire.

L'objet du nouveau médicament est de neutraliser ces dérivés soufrés par de l'extrait de levure et de créer un médicament pour lavage nasal ou auriculaire ou pour application dermique dans une crème.

Par exemple, une solution de Na₂S à 400 mg/litre a un pH de 10,8 et une forte odeur de H₂S. Si on ajoute à un litre de cette solution 5g d'extrait de levure parfaitement soluble, on obtient un pH de 7,1 et une odeur pratiquement neutre. L'extrait de levure est par exemple USP.

Conformément à l'invention, le médicament est constitué par :

| | |
|---|---|
| Na₂S, 9H₂O | 4 mg |
| Extrait de levure | 50 mg |
| Polysorbate 80 | 10 mg |
| Essence de Neroli | 2 µl |
| Solution de NaCl à 9 0/00 qsp | 10 ml |
| pH | 7,3 |
| Odeur | Neroli |

Afin de montrer l'efficacité de ce médicament nouveau, il a été élaboré deux solutés nasaux :

l'un avec de l'extrait de levure (marqué au tableau suivant par A) :

| A | |
|---|---|
| Na₂S, 9H₂O | 4 mg |
| Extrait de levure | 50 mg |
| Polysorbate 80 | 10 mg |
| Essence de Neroli | 2 µl |
| Solution de NaCl à 9 0/00 qsp | 10 ml |
| pH | 7,3 |
| Odeur | Neroli |

et l'autre sans extrait de levure (marqué au tableau suivant par B) :

| B | |
|---|---|
| Na₂S, 9H₂O | 4 mg |
| Extrait de levure | 0 |
| Polysorbate 80 | 10 mg |
| Essence de Neroli | 2 µl |
| Solution de NaCl à 9 0/00 qsp | 10 ml |
| pH | 10,8 |
| Odeur | Hydrogène sulfuré |

Après vérification, on constate que le produit A a un pH neutre et une odeur agréable.

A titre indicatif, pour étudier l'activité du produit A, on l'a comparé à une solution de NaCl à 9 0/00 dont l'activité est connue et qui est largement utilisée en lavage nasal.

50 adultes présentant une rhino-pharyngite ont été divisés en deux groupes de 25. Un groupe a effectué deux lavages par jour avec le produit A, l'autre groupe a effectué deux lavages par jour avec la solution de NaCl. Les deux traitements ont été effectués pendant une période de 3 mois. Aucune autre thérapeutique n'a été administrée pendant cette période.

| Tableau des résultats | | |
|---|---|---|
| | Produit A | Sol. de NaCl |
| Rhinorrhée | | |
| Amélioration | 91,7 % | 59,1 % |

| Oedème de la muqueuse | | |
|---|---|---|
| Amélioration | 94,7 % | 55,6 % |

| Erythème de la muqueuse | | |
|---|---|---|
| Amélioration | 95,8 % | 76 % |

| Tolérance | | |
|---|---|---|
| Parfaite | 76 % | 72 % |
| Bonne | 16 % | 20 % |

En résumé, on peut conclure que l'activité des dérivés soufrés est parfaitement connue mais leur utilisation est très difficile dans le cas d'une application dermique. Le médicament, objet de la présente invention, est donc actif et parfaitement toléré.

Bien que l'exemple précédent soit une solution, il est possible de présenter le nouveau médicament sous la forme d'une poudre soluble, d'une pommade, ces produits étant toujours destinés à un contact dermique.

## Revendications

1. Médicament à base de dérivés soufrés neutralisés, caractérisé en ce qu'il est formé une association neutralisante d'un sulfure par de l'extrait de levure, par exemple U.S.P.

2. Médicament selon la revendication 1, caractérisé en ce que le médicament est constitué par :
| | |
|---|---|
| Na₂S, 9H₂O | 4 mg |
| Extrait de levure | 50 mg |
| Polysorbate 80 | 10 mg |
| Essence de Neroli | 2 µl |
| Solution de NaCl à 9 0/00 qsp | 10 ml |
| pH | 7,3 |
| Odeur | Neroli |

3. Médicament selon la revendication 1, caractérisé en ce que le sulfure est un sulfure de sodium, de potassium, de zinc, de sélénium, et d'une manière plus générale, l'anion S⁻⁻ lié à un cation.

4. Médicament selon l'une des revendications 1 à 3, caractérisé en ce qu'il est présenté sous la forme d'une solution, d'une poudre ou d'une pommade, toujours destiné à un contact dermique.

## Claims

1. Medication based on neutralized sulfur derivatives, characterized in that it is formed of a neutralizing association of a sulfide with a yeast extract, for example a U.S.P. yeast extract.

2. Medication as set forth in claim 1, characterized in that the medication is made of :
| | |
|---|---|
| Na₂S, 9H₂O | 4 mg |
| Yeast extract | 50 mg |
| Polysorbate 80 | 10 mg |
| Neroli essential oil | 2 µl |
| 9 0/00 NaCl solution Q.S. for | 10 ml |
| pH | 7.3 |
| Smell | Neroli |

3. Medication according the claim 1, characterized in that the sulfide is a sulphide of sodium, potassium, zinc, selenium, and more generally the S⁻⁻ anion linked to a cation.

4. Medication according to one of claims 1 to 3, characterized in that it is presented in the form of a solution, a powder or an ointment, always intended for a contact with the skin.

## Patentansprüche

1. Medikament auf der Basis neutralisierte Schwefelderivate, **dadurch gekennzeichnet**, daß es aus einer eine Schwefelverbindung neutralisierenden Verbindung aus Hefeextrakt gebildet ist, z. B. U.S.P.

2. Medikament nach Anspruch 1, **dadurch gekennzeichnet**, daß das Medikament besteht aus:
| | |
|---|---|
| Na₂S, 9H₂O | 4 mg |
| Hefeextrakt | 50 mg |
| Polysorptiv 80 | 10 mg |
| Neroliessenz | 2 µl |
| Kochsalzlösung mit 9 0/00 qsp | 10 ml |
| pH | 7,3 |
| Geruch | Neroli |

3. Medikament nach Anspruch 1, **dadurch gekennzeichnet**, daß die Schwefelverbindung ein Natriumsulfid, Kaliumsulfid, Zinksulfid, Selensulfid und in allgemeinerer Ausdrucksweise das an ein Kation gebundene Anion S⁻⁻ ist.

4. Medikament nach einem der Ansprüche 1 bis 3, **dadurch gekennzeichnet**, daß es in der Form einer Lösung, eines Puders oder einer Pomade dargereicht wird und immer für den Hautkontakt bestimmt ist.
